# EUROPEAN PATENT APPLICATION

(11) **EP 3 858 821 A1**
(43) Date of publication of application: **04.08.2021**
(21) Application number: 20154458.2
(22) Date of filing: 30.01.2020
(51) Int. Cl.: C07D 307/33

(54) **METHOD OF PRODUCING GAMMA-VALEROLACTONE FROM LEVULINIC ACID COMPOUND AND CATALYST USING THEREOF**

(71) Applicant: Clariant Catalysts (Japan) K.K., Tokyo 113-0021 (JP); National University Corporation Chiba University, Chiba-shi, Chiba 263-8522 (JP)
(72) Inventor: YANASE, Daichi, Chiba, 263-8522 (JP); SATO, Satoshi, Chiba, 263-8522 (JP)
(74) Representative: Klingelhöfer, Stefan

(57) **Abstract**

An objective of the present invention is to provide a method of manufacturing gamma-valerolactone (GVL) from levulinic acid compound, and a hydrogenation catalyst used therein. The present invention relates to a method of producing gamma-valerolactone (GVL), the method comprises a step of: bringing a levulinic acid compound into contact with hydrogen in the presence of a hydrogenation catalyst, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).

## Description

### FIELD OF INVENTION

This invention relates to a method of manufacturing gamma-valerolactone (GVL) from a levulinic acid compound, and a hydrogenation catalyst using therein.

### BACKGROUND

Gamma-valerolactone (GVL), a bio-derived material, is expected to be utilized in the production of various kinds of organic compounds such as polymers, olefin compounds, and adipate esters. GVL, which is also called 5-methylbutyrolactone or 5-valerolactone, is produced from a levulinic acid compound. The conversion of a levulinic acid (LA) compound to GVL requires hydrogenation reaction using a catalyst.

Rei Yoshida, Daolai Suna Yasuhiro Yamada, Satoshi Sato, Graham J. Hutchings (2017) "Vapor-phase hydrogenation of levulinic acid to γ-valerolactone over Cu-Ni bimetallic catalysts", Catalysis Communications, volume 97, Pages 79-82 discloses a SiO2-supported Cu-Ni bimetallic catalysts for hydrogenation of LA to GVL.

### SUMMARY OF THE INVENTION

An objective of the invention is to provide a method of manufacturing GVL from a levulinic acid compound, and a hydrogenation catalyst used therein.

An aspect of the invention relates to a method of producing GVL, the method comprises a step of: bringing a levulinic acid compound into contact with hydrogen in the presence of a hydrogenation catalyst, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).

Another aspect of the invention relates to a hydrogenation catalyst for the hydrogenation of a levulinic acid compound, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).

Another aspect of the invention relates to a reactor for the hydrogenation of a levulinic acid compound, the reactor is charged with a hydrogenation catalyst, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).

The method of effectively producing GVL from a levulinic acid compound, and a hydrogenation catalyst used therein is provided.

### DETAILED DESCRIPTION OF THE INVENTION

### Method of producing GVL

The method of producing gamma valerolactone (GVL) comprises a step of bringing a levulinic acid compound into contact with hydrogen in the presence of a hydrogenation catalyst. Gamma valerolactone (GVL) is an organic compound with the structural formula below.

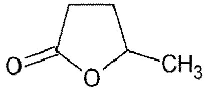

GVL can be produced from a levulinic acid compound by a hydrogenation reaction. The levulinic acid compound is represented by the following formula (1), 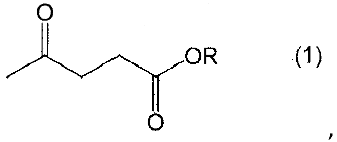 wherein R is hydrogen or an alkyl group of 1 to 6 carbon atoms. In one embodiment the alkyl group of 1 to 6 carbon atoms is selected from the group consisting of methyl group, ethyl group, n-propyl group, n-butyl group, n-pentyl group, n-hexyl group, isopropyl group, isobutyl group, tert-butyl group and sec-butyl group. In another embodiment, R is hydrogen. In one embodiment the levulinic acid compound is a biomass-derived levulinic acid compound, for example lignocellulosic biomass. In this case , GVL is also a biomass-derived material.

The reaction of the levulinic acid compound with hydrogen is carried out in a reactor. There is no limitation on the type of reactor.

In one embodiment a reactor in which the hydrogenation reaction of levulinic acid compound with hydrogen is performed is fully or partially charged with the hydrogenation catalyst. In one embodiment an inert solid such as glass wool or glass beads can be also charged together with the hydrogenation catalyst as an infill.

In one embodiment, the method of producing GVL comprises the steps of: providing a reactor charged with the hydrogenation catalyst, and bringing a levulinic acid compound into contact with hydrogen in the presence of the hydrogenation catalyst by supplying the levulinic acid compound and a hydrogen gas into the reactor. In another embodiment, the method of producing GVL comprises the steps of: providing a reactor charged with the hydrogenation catalyst and a hydrogen gas, and bringing a levulinic acid compound into contact with hydrogen in the presence of the hydrogenation catalyst by supplying the levulinic acid compound into the reactor. In another embodiment, the method of producing GVL comprises the steps of: providing a reactor charged with a hydrogenation catalyst and a levulinic acid compound, and bringing the levulinic acid compound into contact with hydrogen in the presence of the hydrogenation catalyst by supplying a hydrogen gas in the reactor. In another embodiment, the method of producing GVL comprises the steps of: providing the reactor charged with a hydrogen gas and a levulinic acid compound, and bringing the levulinic acid compound into contact with hydrogen in the presence of the hydrogenation catalyst by supplying the hydrogenation catalyst into the reactor.

In one embodiment the levulinic acid compound could be mixed with a solvent to become a levulinic acid compound solution. The levulinic acid compound solution could ease supply of the levulinic acid compound and improve stirrability or flowability. The type of the solvent to dissolve the levulinic acid compound is not limited so long as it does not inhibit the hydrogenation reaction. In one embodiment, the levulinic acid compound solution comprises a solvent selected from the group consisting of water, alcohols, hydrocarbons, amides, ethers, halogenated hydrocarbons. In one embodiment the alcohol is selected from the group consisting of methanol, ethanol, n-propyl alcohol, isopropyl alcohol, n-butyl alcohol, tert-butyl alcohol, ethylene glycol and a mixture thereof. In one embodiment the hydrocarbon is selected from the group consisting of heptane, hexane, cyclohexane, toluene and a mixture thereof. In one embodiment the amide is selected from the group consisting of N,N-dimethylformamide, N,N-dimethylacetamide, N-methyl-2-pyrrolidone and a mixture thereof. In one embodiment the ether is selected from the group consisting of diethyl ether, diisopropyl ether, 1,2-dimethoxyethane, 1,2-diethoxyethane, diethylene glycol dimethyl ether, diethylene glycol diethyl ether, tetrahydrofuran, dioxane and a mixture thereof. In one embodiment the halogenated hydrocarbon is selected from the group consisting of methylene chloride, dichloroethane, chlorocyclohexane and a mixture thereof. In one embodiment the solvent is selected from the group consisting of water, alcohols, hydrocarbons, ethers and a mixture thereof. In one embodiment the solvent comprises water. In one embodiment the content of the solvent in the solution is 1 to 90 wt. %, 10 to 70 wt. % in another embodiment based on the weight of the levulinic acid compound solution.

In another embodiment the levulinic acid compound comprises no solvent.

In one embodiment the solvent and the levulinic acid compound are separately charged or supplied into the reactor.

In one embodiment the levulinic acid compound or the levulinic acid compound solution is in a phase selected from the group consisting of a liquid, a gas and a mixture thereof. In one embodiment the levulinic acid compound solution is in the liquid phase.

The levulinic acid compound or the levulinic acid compound solution in liquid phase can be supplied into the reactor followed by vaporizing by heating the reactor. In another embodiment the levulinic acid compound or the levulinic acid compound solution in the liquid phase can be vaporized by heating before being supplied into the reactor.

In one embodiment the hydrogen is present as a mixture of hydrogen and an inert gas, like nitrogen or a noble gas like Helium or Argon, in another embodiment pure hydrogen gas is used.

The conditions of the hydrogenation reaction of the levulinic acid compound, for example temperature and pressure, can be adjusted to optimize the reaction rate or suppression of side products. In one embodiment the reaction temperature is in the range of from 100 to 400°C., of from 120 to 350°C. in another embodiment, of from 180 to 310°C. in another embodiment, of from 200 to 290°C. in another embodiment. The reaction temperature is the temperature of the hydrogenation catalyst and could be measured with a thermocouple brought into contact with the catalyst or with the gaseous or liquid medium surrounding the catalyst.

In one embodiment the pressure is in a range of from 0.01 to 1.0 MPa, of from 0.04 to 0.7 MPa in another embodiment, of from 0.06 to 0.5 MPa in another embodiment, of from 0.08 to 0.3 MPa in another embodiment. The reaction pressure could be measured with a pressure gauge equipped in the reactor.

In one embodiment, the method of producing GVL comprises the steps of: providing a levulinic acid compound, hydrogen and a hydrogenation catalyst, and bringing the levulinic acid compound into contact with the hydrogen in the presence of the hydrogenation catalyst.

### Catalyst

The hydrogenation catalyst comprises an alumina support of 100 parts by weight and a supported metal comprising 1 to 60 parts by weight of copper (Cu) and 5 to 70 parts by weight of nickel (Ni).

In one embodiment the weight ratio of the supported metal and the alumina support (metal/support) is in a range of from 0.01 to 1.5, of from 0.08 to 1.0 in another embodiment, of from 0.12 to 0.5 in another embodiment, of from 0.18 to 0.3 in another embodiment.

The alumina support is a support comprising alumina (Al₂O₃). In one embodiment the alumina support comprises alumina and an additional inorganic material. In one embodiment the additional inorganic material is selected from the group consisting of silica (SiO₂), ceria (CeO₂), magnesia (MgO), lime (CaO), titania (TiO₂), zirconia (ZrO₂), activated carbon, zeolite and a mixture thereof. In one embodiment the content of the additional inorganic material is 50 weight % (wt. %) or less based on the weight of the support. In another embodiment the alumina support consists of alumina.

In one embodiment the content of alumina (Al₂O₃) in the hydrogenation catalyst is in the range of from 43 to 96.1 wt. %, of from 51 to 95 wt. % in another embodiment, of from 62 to 92 wt. %, of from 69 to 89 wt. % in another embodiment, of from 75 to 85 wt. % in another embodiment, based on the weight of the hydrogenation catalyst.

The supported metal comprises copper and nickel.

In one embodiment the content of copper is in the range of from 1 to 48 parts by weight, of from 2 to 32 parts by weight in another embodiment, of from 3 to 25 parts by weight in another embodiment, of from 4 to 18 parts by weight in another embodiment, of from 5 to 10 parts by weight in another embodiment, when the alumina support is 100 parts by weight.

In one embodiment the content of copper is in the range of from 0.9 to 36 weight % (wt. %), of from 1.2 to 30 wt. % in another embodiment, of from 2.5 to 25 wt. % in another embodiment, of from 3.5 to 18 wt. % in another embodiment, of from 4.2 to 10 wt. % in another embodiment, based on the weight of the hydrogenation catalyst.

In one embodiment the content of nickel is in the range of from 5 to 58 parts by weight, of from 6 to 42 parts by weight in another embodiment, of from 8 to 31 parts by weight in another embodiment, of from 9 to 27 parts by weight in another embodiment, of from 12 to 21 parts by weight in another embodiment, when the alumina support is 100 parts by weight.

In one embodiment the content of nickel is in the range of from 3 to 41 wt. %, of from 4 to 36 wt. % in another embodiment, of from 6 to 29 wt. % in another embodiment, of from 8.5 to 24 wt. % in another embodiment, of from 10 to 20 wt. % in another embodiment, based on the weight of the hydrogenation catalyst.

In one embodiment the weight ratio of Cu and Ni (Cu:Ni) is 1:1 to 1:5, 1:1.2 to 1:4.2 in another embodiment, 1:1.5 to 1:3.8 in another embodiment, 1:2.0 to 1:3.0 in another embodiment.

In one embodiment the weight ratio of the sum of Cu and Ni content and the alumina support content [(Cu+Ni)/Al₂O₃ support] is 0.01 to 1.5, 0.08 to 1.0 in another embodiment, 0.12 to 0.5 in another embodiment, 0.18 to 0.3 in another embodiment.

In one embodiment the supported metal further comprises one or more other additional metal other than copper and nickel. The additional metal is selected from the group consisting of Li, Na, K, Rb, Mg, Ca, Sr, Ba, Y, Ti, Zr, V, Nb, Cr, Mo, W, Mn, Fe, Ru, Os, Co, Rh, Ir, Pd, Pt, Ag, Au, Zn, Al, Sn, Bi and a mixture thereof in an embodiment, The additional metal is selected from the group consisting of Ru, Rh, Pd and a mixture thereof in another embodiment. In one embodiment the content of the additional metal is 10 wt. % or less based on the weight of the supported metal. In one embodiment the supported metal consists of copper and nickel.

In one embodiment the hydrogenation catalyst consists of an alumina support and an supported metal consisting of copper and nickel.

In one embodiment the particle diameter D50 of the hydrogenation catalyst is in the range of from 50 to 5000 µm, of from 80 to 3000 µm in another embodiment, of from 100 to 2500 µm in another embodiment, of from 120 to 1000 µm in another embodiment, of from 200 to 800 µm in another embodiment.

In one embodiment the surface area (S_{BET}) of the hydrogenation catalyst is in the range of from 10 to 1000 m²g⁻¹, of from 32 to 800 m²g⁻¹ in another embodiment, of from 58 to 645 m²g⁻¹ in another embodiment, of from 68 to 500 m²g⁻¹ in another embodiment, of from 80 to 320 m²g⁻¹ in another embodiment.

In one embodiment the hydrogenation catalyst is porous. In another embodiment it has a pore volume in the range of from 0.01 to 5 cm³g⁻¹, of from 0.1 to 3.5 cm³g⁻¹, of from 0.25 to 2.7 cm³g⁻¹ in another embodiment, of from 0.38 to 1.8 cm³g⁻¹ in another embodiment, of from 0.41 to 1.2 cm³g⁻¹ in another embodiment.

In one embodiment the average pore diameter of the hydrogenation catalyst is in the range of from 1 to 100 nm, of from 5 to 85 nm in another embodiment, of from 10 to 72 nm in another embodiment, of from 12 to 58 nm in another embodiment, of from 15 to 38 nm in another embodiment.

The particle diameter D50 was measured with a laser diffraction scattering particle size distribution analyzer of the Microtrac MT3000II series from MicrotracBEL Corp. The surface area, the pore volume and the average pore diameter were determined with an automatic specific surface area/pore size distribution measurement instrument of the type BELSORP-mini-X from MicrotracBEL Corp.

The particle diameter, surface area, pore volume and average pore diameter can be adjusted in consideration of reaction rate and strength of the hydrogenation catalyst.

The shape of the hydrogenation catalyst is not limited. It can be selected in consideration of particle diameter and strength of the catalyst, the reaction system and type and shape of a reactor, for example. In one embodiment, the shape of the hydrogenation catalyst is selected from the group consisting of powder, granules, pellets, spherical ball, tablet, cylinder and a mixture thereof.

### Method of Manufacturing the Catalyst

In one embodiment the catalyst is manufactured with an impregnation method. In one embodiment the support is impregnated with a solution containing at least Cu and Ni. In one embodiment the solution is an aqueous solution comprising a Cu nitrate and/or a Ni nitrate. In another embodiment the Cu and Ni compound are applied to the support by use of separate solutions, where the alumina support is impregnated with either the Cu containing solution or the Ni containing solution first followed by impregnation with the solution comprising the other Cu or Ni compound. In one embodiment the Cu nitrate is Cu(NO₃)₂·3H₂O. In one embodiment the Ni nitrate is Ni(NO₃)₂·6H₂O.

In the following the term "solution" can mean both, either the Cu and Ni containing solution or the Cu containing solution or the Ni containing solution.

The solution can additionally comprise citric acid. In another embodiment the solution comprises no citric acid.

In one embodiment the solution temperature is in the range of from 10 to 80°C.. The impregnation is performed by immersing the support in the at least one solution or dropping the at least one solution onto the support. In one embodiment the immersion time is in the range of from 1 to 30 hours.

After impregnation, the alumina support is optionally dried by heating at a temperature in the range of from 30 to 200°C.. In one embodiment the drying time is in the range of from 1 to 30 hours. During drying water may evaporate and being removed from the impregnated catalyst. In one embodiment the support is subjected to calcination at a temperature in the range of from 100°C. to 600°C., of from 250°C. to 400°C. in another embodiment. The support is calcined in a furnace at the setting temperature of from 150°C. to 700°C, of from 200°C. to 550°C in another embodiment, of from 250°C. to 400°C. in another embodiment. In one embodiment the calcination time is in the range of from 1 to 10 hours, of from 2 to 8 hours in another embodiment. After calcination the obtained material can be used as a hydrogenation catalyst.

In one embodiment the hydrogenation catalyst is further subjected to a reduction treatment. The hydrogenation catalyst is typically reduced by contact with hydrogen.

In one embodiment, a method of manufacturing the hydrogenation catalyst comprises the steps of: impregnating a support comprising alumina with at least one solution comprising nickel and/or copper to form a precatalyst, and calcining the precatalyst.

### Reactor

One further aspect of the invention concerns a reactor for the hydrogenation of a levulinic acid compound, wherein the reactor is charged with a hydrogenation catalyst, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).

### EXAMPLES

### Example 1

The hydrogenation catalyst was prepared by an impregnation method as follows. An aqueous solution of Cu and Ni was prepared by dissolving 2.85 g of Cu(NO₃)₂·3H₂O and 8.67 g of Ni(NO₃)₂·6H₂O in 125 g of water at 40°C. This aqueous solution was applied by dropping the solution in form of drops of 0.3 cm³ in volume onto a support of Al₂O₃ (10 g of JRC-ALO-6, Reference Catalyst Division, the Catalysis Society of Japan) followed by heating at 70°C. for water evaporation to some extent. The drop and heating steps were repeated until the aqueous solution was used up. The Al₂O₃ support had an average particle diameter D50 of 300 µm and a surface area (S_{BET}) of 180 m²g⁻¹. The resulting sample was dried in an oven at 110°C. for 12 hours. After the drying step, the sample was calcined in a furnace at a setting temperature of 300°C. for 3 hours to form a catalyst. The catalyst (Cu-Ni/Al₂O₃) obtained above contained 100 parts by weight of the Al₂O₃ support, 7.5 parts by weight of Cu and 17.5 parts by weight of Ni as the supported metal. The catalyst had a surface area (S_{BET}) of 117 m²g⁻¹, a pore volume of 0.68 cm³g⁻¹, and an average pore diameter of 23.5 nm. The BET surface area, Pore volume and Average pore diameter were measured by using an automatic specific surface area pore size distribution measurement instrument (BELSORP-mini X, MicrotracBEL Corp.) after pretreatment of drying with BELPREP-vac-II from MicrotracBEL Corp.

### Application Example 1

The hydrogenation reaction of levulinic acid was carried out as follows. A vapor-phase hydrogenation of the levulinic acid was performed in a fixed bed flow-type reactor. The reactor contained a reaction tube of cylindrical shape (17 mm diameter, 50 mm long) with an inlet and an outlet. 0.25 gram of the catalyst was placed on a glass wool bed stuffed in the reaction tube. The catalyst was reduced with pure hydrogen gas (H₂) at 300°C. for one hour. The pure levulinic acid vaporized was fed together with a gas of pure hydrogen at a supply rate of 30 cm³min⁻¹ into the reaction tube through the inlet at a supply rate of 1.65 g h⁻¹. The temperature around the catalyst measured with a thermocouple was 250°C. The pressure inside the reaction tube was 0.1 MPa. The gas of the outlet was directed to a dry ice-acetone trap of -78°C. to collect the reaction mixture liquid.

The remaining levulinic acid (LA) amount and the generated GVL amount in the reaction mixture was measured to determine the LA conversion rate as well as the catalyst's GVL selectivity. The collected reaction mixture was quantitatively analyzed by gas chromatography (GC-14B, Shimadzu, Japan) equipped with a flame ionization detector and a capillary column of InertCap® 1 (inner diameter 0.25 mm, 60 m long, GL-Science, Japan). The LA conversion (mol %) was calculated by subtracting the remaining amount of levulinic acid in the outlet gas (LA remaining) from the amount of levulinic acid in the inlet gas (LA initial) followed by dividing by the LA initial, multiplied with 100 ([(LA initial-LA remaining)/LA initial]x100). The GVL selectivity was calculated by dividing the amount of generated GVL by the difference of the LA initial and the LA remaining, multiplied with 100 ([GVL/(LA initial-LA remaining)]x100). The measurement was carried out 5, 15, 30, and 60 hours after start of the reaction.

### Comparative Example 1

The catalyst was prepared with the same method as Example 1 except using a support of SiO₂ (CARiACT Q10, Fuji Silycia Chemical Ltd.) having an average particle diameter of 290 µm, a surface area (S_{BET}) of 295 m²g⁻¹, a pore volume of 1 cm³g⁻¹. The obtained catalyst (Cu-Ni/SiO₂) contained 100 parts by weight of the SiO₂ support, 7.5 parts by weight of copper and 17.5 parts by weight of nickel as the supported metal. The obtained catalyst had a surface area (S_{BET}) of 200 m²g⁻¹, a pore volume of 0.86 cm³g⁻¹, and an average pore diameter of 17.4 nm.

### Comparative Example 2

The catalyst was prepared with the same method as Example 1 except using an aqueous solution of Cu(NO₃)₂·3H₂O without Ni(NO₃)₂·6H₂O. The obtained catalyst (Cu/Al₂O₃) contained 100 parts by weight of the Al₂O₃ support and 25 parts by weight of copper as the supported metal. The obtained catalyst had a surface area (S_{BET}) of 213 m²g⁻¹, a pore volume of 0.76 cm³g⁻¹, and an average pore diameter of 14.4 nm.

### Comparative Example 3

The catalyst was prepared with the same method as Example 1 except for using an aqueous solution of Ni(NO₃)₂·6H₂O without Cu(NO₃)₂·3H₂O and the reduction temperature of the catalyst was 400°C., which was necessary to reduce the sole oxidized nickel compound. The obtained catalyst (Ni/Al₂O₃) contained 100 parts by weight of the Al₂O₃ support and 25 parts by weight of nickel as the supported metal. The obtained catalyst had a surface area (S_{BET}) of 126 m²g⁻¹, a pore volume of 0.78 cm³g⁻¹, and an average pore diameter of 25.2 nm.

### Results

The LA conversion rate and the selectivity are shown in Table 1 and 2 respectively. The LA conversion of Cu-Ni/Al₂O₃ (Example 1) was kept sufficiently above 90 mol % with a high selectivity above 97 mol % for a period of 60 hours . To the contrary, Comparative Example 1 (Com. Ex. 1) showed that the LA conversion rate went down to 68 mol % after 30 hours, while for Comparative Example 2 the LA conversion rate even went down below 20 mol% in the first 5 hours. Comparative Example 3 showed a GVL selectivity of just 89 mol % within the first 5 hours although the LA conversion rate was 93 % or higher for 30 hours.

**Table 1**

| | | Conversion (mol %) | | | |
|---|---|---|---|---|---|
| | Catalyst | 5 h | 15 h | 30 h | 60 h |
| Example 1 | Cu-Ni/Al₂O₃ | 100 | 99 | 99 | 92 |
| Com. Ex. 1 | Cu-Ni/SiO₂ | 99 | 86 | 68 | - |
| Com. Ex. 2 | Cu/Al₂O₃ | 16 | - | - | - |
| Com. Ex. 3 | Ni/Al₂O₃ | 100 | 100 | 93 | - |

**Table 2**

| | | Selectivity (mol %) | | | |
|---|---|---|---|---|---|
| | Catalyst | 5 h | 15 h | 30 h | 60 h |
| Example 1 | Cu-Ni/Al₂O₃ | 97 | 97 | 98 | 99 |
| Com. Ex. 1 | Cu-Ni/SiO₂ | 100 | 99 | 96 | - |
| Com. Ex. 2 | Cu/Al₂O₃ | 84 | - | - | - |
| Com. Ex. 3 | Ni/Al₂O₃ | 89 | 89 | 89 | - |

## Claims

1. A method of producing gamma-valerolactone (GVL), the method comprises a step of:
bringing a levulinic acid compound into contact with hydrogen in the presence of a hydrogenation catalyst, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).

2. The method of claim 1, wherein the levulinic acid compound is represented by the following formula (1), 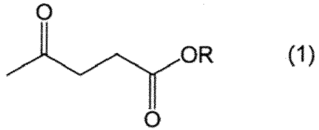 wherein R represents a hydrogen atom or an alkyl group of 1 to 6 carbon atoms.

3. The method of claim 1 or 2, wherein the reaction temperature around the hydrogenation catalyst is in the range of from 100 to 400°C.

4. A hydrogenation catalyst for the hydrogenation of a levulinic acid compound, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).

5. The hydrogenation catalyst of claim 4, wherein the weight ratio of the supported metal and the alumina support (metal/support) is in the range of from 0.01 to 1.5.

6. The hydrogenation catalyst of claim 4 or 5, wherein the weight ratio of copper and nickel (Cu:Ni) is 1:1 to 1:5.

7. The hydrogenation catalyst of any of the claims 4 to 6, wherein the particle diameter D50 of the hydrogenation catalyst is in the range of from 50 to 5000 µm.

8. The hydrogenation catalyst of any of the claims 4 to 7, wherein the specific surface area (S_{BET}) of the hydrogenation catalyst is in the range of from 10 to 1000 m²g⁻¹.

9. The hydrogenation catalyst of any of the claims 4 to 8, wherein the pore volume of the hydrogenation catalyst is in the range of from 0.01 to 5 cm³g⁻¹.

10. The hydrogenation catalyst of any of the claims 4 to 9, wherein the content of copper is in the range of from 0.9 to 36 wt. %, based on the weight of the hydrogenation catalyst.

11. The hydrogenation catalyst of any of the claims 4 to 10, wherein the content of nickel is in the range of from 3 to 41 wt., based on the weight of the hydrogenation catalyst.

12. The hydrogenation catalyst of any of the claims 4 to 11, wherein the supported metal further comprises an additional metal selected from the group consisting of Li, Na, K, Rb, Mg, Ca, Sr, Ba, Y, Ti, Zr, V, Nb, Cr, Mo, W, Mn, Fe, Ru, Os, Co, Rh, Ir, Pd, Pt, Ag, Au, Zn, Al, Sn, Bi and a mixture thereof.

13. A reactor for the hydrogenation of a levulinic acid compound, wherein the reactor is charged with a hydrogenation catalyst, wherein the hydrogenation catalyst comprises an alumina support (Al₂O₃) of 100 parts by weight and a supported metal comprising 1 to 60 parts by weight of copper (Cu) and 5 to 70 parts by weight of nickel (Ni).
